## Europäisches Patentamt
## European Patent Office
## Office européen des brevets

(19)

(11) Veröffentlichungsnummer: **0 271 814**
**B1**

(12) # EUROPÄISCHE PATENTSCHRIFT

(45) Veröffentlichungstag der Patentschrift:
07.11.90

(51) Int. Cl.⁵: **B01J 23/66**, C07D 301/10

(21) Anmeldenummer: **87118197.0**

(22) Anmeldetag: **09.12.87**

(54) **Silberkatalysator, seine Herstellung und Verwendung.**

(30) Priorität: **18.12.86 DE 3643248**

(43) Veröffentlichungstag der Anmeldung:
**22.06.88 Patentblatt 88/25**

(45) Bekanntmachung des Hinweises auf die Patenterteilung:
**07.11.90 Patentblatt 90/45**

(84) Benannte Vertragsstaaten:
**BE DE GB NL**

(56) Entgegenhaltungen:
**EP-A- 0 172 565**
**EP-A- 2 111 397**
**US-A- 4 368 144**

(73) Patentinhaber: **BASF Aktiengesellschaft,
Carl-Bosch-Strasse 38, D-6700 Ludwigshafen(DE)**

(72) Erfinder: **Boehning, Karl-Heinz, Dr.,
Walther-Rathenau-Strasse 32, D-6100 Darmstadt(DE)**
Erfinder: **Mross, Wolf Dieter, Dr.,
Anselm-Feuerbach-Strasse 21, D-6710 Frankenthal(DE)**
Erfinder: **Schwarzmann, Matthias, Dr.,
Carl-Bosch-Strasse 54, D-6703 Limburgerhof(DE)**
Erfinder: **Becker, Hans-Juergen, Dr.,
Bergsteinstrasse 36, D-6730 Neustadt(DE)**
Erfinder: **Plueckhan, Juergen, Dr., Bensheimer Ring 19 b,
D-6710 Frankenthal(DE)**
Erfinder: **Renner, Klaus-Christian, Dr.,
Carl-Spitzweg-Strasse 2, D-6710 Frankenthal(DE)**

## Beschreibung

Es ist bekannt Silber enthaltende Katalysatoren für die Direktoxidation von Ethylen mit Sauerstoff zu Ethylenoxid zu verwenden. Zumeist dient $\alpha$-Aluminiumoxid als Träger für die Herstellung der Silberkatalysatoren. Es sind verschiedene Verfahren für die Aufbringung der katalytisch aktiven Komponente Silber auf den Träger bekannt (US-PS 2 294 383, 3 423 328, 3 172 893).

Aus EP-A 172 565 ist ein Silberkatalysator mit einem schichtartigen Aufbau bekannt, in dem im Hinblick auf die äußerste und innerste Katalysatorschicht ein spezieller Gradient bezüglich deren Beladung mit Silber und den Alkalimetallpromotoren besteht. Neben den Alkalimetallpromotoren kann dieser Katalysalor auch noch u.a. Calcium enthalten, welches auf das Trägermaterial aufgebracht wird. Der spezielle Verteilungsgradient des Silbers und der Alkalimetallpromotoren von der äußeren zur inneren Katalysatorschicht wird durch eine besondere Tempertechnik, nämlich die Temperung mit überhitztem Wasserdampf, erzeugt.

Die Angaben über die wünschenswerten Eigenschaften die das Trägermaterial aufweisen soll, gehen in der Literatur auseinander. In aller Regel werden deshalb in Patentschriften recht weite Bereiche für die physikalischen Eigenschaften des Trägers beansprucht.

So werden in DE-OS 3 010 533 Oberflächen von weniger als 1 m²/g und Porenvolumina von 0,2 bis 0,6 cm³/g bei einem Porendurchmesser zwischen 500 und 50 000 nm angegeben. Die chemische Zusammensetzung des Trägers wird als nicht entscheidend bezeichnet. Als mögliches Schüttgewicht werden Werte von 700 kg/m³ angegeben.

In DE-OS 2 712 785 werden Träger mit BET-Oberflächen von 0,03 bis 2 m²/g und Porenvolumina von 0,25 bis 0,65 cm³/g als besonders geeignet beschrieben. Zu ähnlichen Aussagen kommt die US-PS 3 962 136.

Geeignete Träger sind z.B. auch aus DE-OS 2 655 738 bekannt. Allerdings werden in den Beispielen keine mit Alkalimetallen dotierten Katalysatoren wie sie die vorliegende Anmeldung beansprucht hergestellt oder untersucht.

Bei diesen Katalysatoren ist aber die Zusammensetzung der Komponenten und die Dotierung nicht optimal. Die Bedeutung der Alkalimetalle als Promotoren ist für sich genommen beispielsweise aus DE-OS 23 00 512 oder DE-OS 2 753 359 bekannt. Das dort verwendete Trägermaterial entspricht aber nicht dem neuesten Stand der Technik.

Im Gegensatz dazu zeigen die jüngsten technischen Erfahrungen daß nur kleine Ausschnitte aus den bekannten Bereichen von Trägern und Promotoren wirklich vorteilhafte Eigenschaften bei den gefertigten Katalysatoren ergeben. Überraschend gute Ergebnisse bei Aktivität, Selektivität und vor allem Standzeit erzielt man bei der Direktoxidation von Ethylen mlt Sauerstoff zu Ethylenoxid mit einem Silberkatalysator auf porösem Träger aus $\alpha$-Aluminiumoxid der bestimmte Mengen an löslichen Calcium-, Aluminium-, Kalium- und Natriumsalzen enthält mit einer BET-Oberfläche von nicht weniger als 0,4 und nicht mehr als 0,8 m²/g, einem Porenvolumen von wenigstens 0,45 ml/g, wobei die Poren gleichermaßen für kaltes und warmes Wasser zugänglich sind, dessen Schüttgewicht 700 kg/m³ nicht überschreitet, und der eine Form hat, die im Reaktor eine geometrische Oberfläche von mindestens 600 m²/m³ realisiert, auf welcher mindestens 13 Gew.-% Silber als aktive Komponente aufgebracht werden und je m³ Reaktor mindestens 110 kg Silber zur Verfügung stehen und die Katalysatoren zusätzlich 100 bis 300 Gew.-ppm Lithium und 200 bis 600 Gew.-ppm Caesium enthalten.

Der für die Wirksamkeit der Katalysators wichtige Träger wird aus Aluminiumoxid gefertigt. Sein Gehalt an $\alpha$-Al₂O₃ soll mindestens 99 Gew.-% betragen. Die Gehalte an salpetersäurelöslichen Calcium- und/oder Aluminiumionenverbindungen können 200 bis 2000 ppm betragen und die Gehalte an löslichen Kalium- und/oder Natriumverbindungen sollen 50 ppm nicht unterschreiten. Das Trägermaterial aus hochreinem $\alpha$-Al₂O₃ soll zweckmäßig eine BET-Oberfläche von nicht weniger als 0,4 m²/g und nicht mehr als 0,8 m²/g aufweisen. Das Porenvolumen des Trägermaterials wird zweckmäßig durch die allgemein bekannte Methode der Wasseraufnahme bzw. der Quecksilberporosimetrie bestimmt. Das durch nur fünfminütiges Einwirken von Wasser bei Raumtemperatur festgestellte Porenvolumen soll sich nur unwesentlich z.B. maximal 10% von dem nach den genannten Methoden bestimmten Volumen unterscheiden.

Die Porenverteilung kann bimodal oder unimodal sein, wobei im bimodalen Fall die größeren Poren vorzugsweise zumindest 50% des Gesamtporenvolumens ergeben und einen mittleren Durchmesser von etwa 10 000 bis 40 000 nm haben die kleineren Poren Durchmesser von etwa 500 bis 2000 nm und im unimodalen Fall der mittlere Porendurchmesser 1000 bis 5000 nm beträgt. Man kann durch Auswahl geeigneter geometrischer Formen der Trägerpartikel das Schüttgewicht des Trägermaterials so einstellen daß es 700 kg/m³ nicht überschreitet und die äußere geometrische Oberfläche des Katalysators pro Reaktorvolumen dabei 600 m²/m³ nicht unterschreitet.

Die katalytisch aktiven Metallkomponenten werden durch ein Trinkverfahren aufgebracht das aus einer oder mehreren Trinkstufen bestehen kann und das eine oder mehrere Temperungsstufen umfaßt. Dabei soll der Katalysatorträger mit mindestens 13 Gew.-% Silber beaufschlagt werden und eine Silbermenge von mindestens 110 kg Silber pro m³ Reaktor erreicht werden; der Gehalt an Lithium soll 100 bis 300 Gew.-ppm und der Gehalt an Caesium 200 bis 600 Gew.-ppm betragen.

Die erfindungsgemäßen Silberkatalysatoren werden für die Herstellung von Ethylenoxid aus Ethylen und Sauerstoff in der Gasphase eingesetzt. Der Vorteil besteht darin daß diese beim Einsatz für die Herstellung von Ethylenoxid bei guter Anfangsselektivität einen nur etwa halb so großen Selektivitätsabfall zeigen wie die bekannten Katalysatoren die Standzeiten demzufolge höher sind. Gleichzeitig erhöht sich die Belastungsflexibilität der Katalysatoren. Auch die Aktivität nimmt zu so daß niedrigere Reaktortemperaturen möglich sind.

Beispiele

Die Herstellung der erfindungsgemäßen und der nicht erfindungsgemäßen Katalysatoren (Vergleichskatalysatoren) auf den verschiedenen Trägern wurde in folgender Weise durchgeführt:

Silbernitrat wird in zweimal soviel Molen sec.-Butylamin gelöst. Dieser Lösung wird je Ansatz für 100 g Träger 1 ml einer wäßrigen Lithiumnitrat-Lösung (beispielsweise 22,75 g $LiNO_3$ auf 100 ml Lösung) zugesetzt. Danach wird durch Zugabe von VE-Wasser das Lösungsvolumen auf das 1,1 fache erhöht.

Entsprechend der zu erwartenden Flüssigkeitsaufnahme des Trägers wird dieser mit der Lösung imprägniert und einen Tag bei Raumtemperatur gelagert. Dann wird der imprägnierte Träger in einen Umluftofen überführt und bei 240°C im Stickstoffstrom getempert bis die Reaktion abgeklungen ist.

In einem zweiten Tränkschritt wird der Katalysator mit einer zur vollständigen Tränkung ausreichenden Menge einer methanolischen Lösung behandelt die 1 Vol.% sec.-Butylamin und 1 ml einer Caesiumhydroxid-Lösung (beispielsweise 5,46 g CsOH auf 100 ml Lösung mit Methanol) enthält. Es schließt sich ein Trocknungsschritt im Stickstoffstrom an.

Die Caesiumlösung (ohne Methanolzusatz) kann alternativ gleichzeitig mit der Silbersalzlösung auf den Träger aufgetränkt werden.

Man kann aber auch die Promotoren Lithium und Caesium vor der Silberabscheidung auf den Träger aufbringen.

Die angegebenen Gehalte an Lithium und Caesium (Tab. 1 und 2) werden für den jeweils eingesetzten Träger in gesonderten Versuchen optimiert.

Beispiel 1

Die obengenannten Katalysatoren werden zerkleinert und jeweils 10 g der Siebfraktion 0,6 bis 0,75 mm werden in einen Reaktor aus rostfreiem Stahl von 5 mm Innendurchmesser gefüllt. Der Reaktor besitzt einen Mantel durch den eine thermostatisierende Flüssigkeit geschickt wird. Durch den Reaktor wird ein Gas der Zusammensetzung: 30% Ethylen, 8% Sauerstoff, 2 ppm Inhibitor, Rest Stickstoff geleitet. Der Druck beträgt 16 bar und die Katalysatorbelastung 3300 Nl Gas/l Kontakt · h. Die Temperatur wird so eingeregelt daß der Sauerstoffumsatz 50% beträgt. Nach 2 Tagen werden Proben gezogen und die Aktivität und Selektivität bestimmt.

Die vorteilhaften reaktionstechnischen Ergebnisse wie sie sich mit den auf den Trägern A und B hergestellten Katalysatoren erzielen lassen, sind in Tabelle 3 aus Mittelwerten verschiedener Messungen zusammengestellt. Die weniger günstigen Ergebnisse mit allen anderen Trägermaterialien (C–K) zeigt der zweite Teil der Tabelle.

Dabei kann man alle drei Varianten der Katalysatortränkung wie oben beschrieben anwenden.

Beispiel 2

Die Katalysatoren A, B, C, F und I werden unzerkleinert in einer Menge von je 13 dm³ in einen stählernen druckfesten Reaktor gefüllt der dem in technischen Anlagen üblichen Einzelrohr entspricht. Der Reaktor besitzt einen Mantel durch den eine thermostatisierende Flüssigkeit geschickt wird. Durch den Reaktor wird ein Gas der Zusammensetzung: 30% Ethylen, 8% Sauerstoff, 6,5% $CO_2$, 4% Argon, 3 ppm Inhibitor und 50% Methan geschickt. Der Druck beträgt 16 bar. Die Temperatur im Kühlmedium des Reaktors wird in einer ersten Versuchsreihe a so eingestellt daß bei einer Raumgeschwindigkeit von 2000 Nm³ Gas/m³ Kontakt · h ein Sauerstoffumsatz von 35% erreicht wird. In einer zweiten Versuchsreihe wird bei einer Raumgeschwindigkeit von 4 000 Nm³ Gas/m³ Kontakt · h 50% des Sauerstoffs umgesetzt. Proben werden jeweils nach einem Monat und nach 12 Monaten gezogen.

Tabelle 4 zeigt Standzeitverhalten und Belastungsflexibilität der eingesetzten Katalysatoren.

Die erfindungsgemäßen Träger haben die in der nachfolgenden Tabelle 1 zusammengestellten Eigenschaften. Vergleichbare bekannte Träger mit in verschiedener Hinsicht abweichenden Eigenschaften sind in Tabelle 2 enthalten. Diese Träger haben nicht die gewünschten vorteilhaften Eigenschaften, wie in den folgenden Beispielen gezeigt wird.

Tabelle 1: Physikalische und chemische Daten der erfindungsgemäßen Träger und Katalysatoren

| Trägerbezeichnung | | A | B |
|---|---|---|---|
| Gehalt an $\alpha$-$Al_2O_3$ | [Gew.%] | 99,8 | 99,1 |
| Gehalt an $SiO_2$ | [Gew.%] | 0,1 | 0,6 |
| Gehalt an mit $HNO_3$ löslichen Ionen | [Gew.-ppm] | | |
| | Al | 250-2000 | 150-2000 |
| | Ca | 150-2000 | 50-2000 |
| | K | 20-1000 | 20-1000 |
| | Na | 50-1000 | 80-1000 |
| BET-Oberfläche | [m²/g] | 0,70 | 0,60 |
| Wasseraufnahme | [ml/g] | | |
| | kalt | 0,45 | 0,42 |
| | kochen | 0,50 | 0,45 |
| Schüttgewicht | [kg/m³] | 620 | 700 |
| spez. Oberfläche | [m²/m³] | 640 | 670 |
| Silberdichte | [kg/m³] | 115 | 120 |
| Silbergehalt | [Gew.%] | 13 | 15 |
| Lithiumgehalt | [Gew.-ppm] | 100 | 250 |
| Caesiumgehalt | [Gew.-ppm] | 450 | 400 |
| Katalysatorbezeichnung | | A | B |

EP 0 271 814 B1

Tabelle 2: Eigenschaften von nicht erfindungsgemäßen Trägermaterialien und Katalysatoren

| | | C | D | E | F | G | H | I | A | B |
|---|---|---|---|---|---|---|---|---|---|---|
| Trägerbezeichnung | | C | D | E | F | G | H | I | A | B |
| Gehalt an $\alpha$-$Al_2O_3$ | [Gew.-%] | 99,8 | 99,5 | 99,8 | 99,8 | 99,8 | 98,5 | 99,6 | | |
| Gehalt an $SiO_2$ | [Gew.-%] | 0,1 | 0,4 | 0,1 | 0,05 | 0,04 | 1,4 | 0,1 | | |
| Gehalt an mit $HNO_3$ löslichen Ionen | [Gew.-ppm] | | | | | | | | | |
| | Al | 250 | 600 | 300 | 100 | 150 | 60 | 130 | | |
| | Ca | 350 | 500 | 400 | 100 | 100 | 10 | 300 | | |
| | K | 20 | 40 | 30 | 5 | 5 | 2 | 10 | | |
| | Na | 30 | 80 | 80 | 60 | 100 | 50 | 20 | | |
| BET-Oberfläche | [$m^2$/g] | 0,45 | 0,50 | 0,50 | 0,35 | 0,35 | 0,35 | 0,25 | | |
| Wasseraufnahme | [ml/g] | | | | | | | | | |
| | kalt | 0,34 | 0,33 | 0,35 | 0,39 | 0,28 | 0,31 | 0,22 | | |
| | kochen | 0,43 | 0,42 | 0,43 | 0,48 | 0,35 | 0,35 | 0,25 | | |
| Schüttgewicht | [kg/$m^3$] | 730 | 730 | 600 | 650 | 810 | 810 | 1050 | | |
| spez. Oberfläche | [$m^2$/$m^3$] | 670 | 670 | 570 | 630 | 630 | 630 | 670 | | |
| Silberdichte | [kg/$m^3$] | 100 | 100 | 90 | 105 | 110 | 110 | 85 | | |
| Silbergehalt | [Gew.-%] | 12 | 12 | 12 | 14 | 10 | 10 | 8 | 13 | 15 |
| Lithiumgehalt | [Gew.-ppm] | 150 | 100 | 200 | 130 | 100 | 150 | 140 | - | 250 |
| Caesiumgehalt | [Gew.-ppm] | 300 | 250 | 350 | 200 | 300 | 150 | 150 | 450 | - |
| Katalysatorbezeichnung | | C | D | E | F | G | H | I | J | K |

Tabelle 3: Ergebnisse der Versuche nach Anwendungsbeispiel 1

| Träger | Selektivität [%] | Aktivität [°C] |
|---|---|---|
| A | 82,3 | 217 |
| B | 82,2 | 218 |
| C | 81,9 | 222 |
| D | 81,9 | 225 |
| E | 81,5 | 227 |
| F | 81,0 | 221 |
| G | 80,9 | 224 |
| H | 80,1 | 230 |
| I | 80,0 | 226 |
| J | 81,0 | 225 |
| K | 76,0 | 210 |

Tabelle 4: Ergebnisse der Versuche nach Anwendungsbeispiel 2

| Träger | Selektivität [%] | Aktivität [%] | Selektivität [%] | Aktivität [%] |
|---|---|---|---|---|
| | bei 35 % $O_2$-Umsatz | | bei 50 % $O_2$-Umsatz | |
| nach 1 Monat | | | | |
| A | 82,5 | 196 | 81,5 | 220 |
| B | 82,5 | 197 | 81,3 | 220 |
| C | 82,1 | 200 | 80,4 | 226 |
| F | 81,5 | 200 | 80,0 | 228 |
| I | 81,0 | 204 | 79,5 | 234 |
| nach 12 Monaten | | | | |
| A | 81,5 | 200 | 80,4 | 228 |
| B | 81,6 | 205 | 80,4 | 230 |
| C | 80,5 | 210 | 79,0 | 238 |
| F | 80,2 | 208 | 78,4 | 242 |
| I | 79,4 | 213 | 77,0 | 249 |

**Patentansprüche**

1. Silberkatalysator mit Lithium und Cäsium als Promotoren für die Direktoxidation von Ethylen mit Sauerstoff zu Ethylenoxid auf einem porösen Träger, der im wesentlichen aus α-Aluminiumoxid besteht und bestimmte Mengen an löslichen Calcium-, Aluminium-, Kalium- und Natriumsalzen enthält, dadurch gekennzeichnet, daß der Träger eine BET-Oberfläche von nicht weniger als 0,4 m²/g und nicht mehr als 0,8 m²/g, ein Porenvolumen von wenigstens 0,45 ml/g, wobei die Poren gleichermaßen für kaltes und warmes Wasser zugänglich sind, ein Schüttgewicht von nicht mehr als 700 kg/m³ und eine Form hat, die im Reaktor eine geometrische Oberfläche von mindestens 600 m²/m³ realisiert, auf welcher mindestens 13 Gew.% Silber als aktive Komponente aufgebracht werden und je m³ Reaktor mindestens 110 kg Silber zur Verfügung stehen und als Promotoren 100 bis 300 Gew.-ppm Lithium und 200 bis 600 Gew.-ppm Cäsium enthalten sind.

2. Silberkatalysator nach Anspruch 1, dadurch gekennzeichnet, daß im Träger salpetersäurelösliche Calcium- und/oder Aluminiumverbindungen in Mengen von 200 bis 2000 ppm und mindestens 50 ppm lösliche Kalium- und/oder Natriumverbindungen enthalten sind.

3. Silberkatalysator nach Anspruch 1 und 2, dadurch gekennzeichnet, daß das Trägermaterial zu mindestens 99% aus hochreinem $\alpha$-Al$_2$O$_3$ besteht.

4. Silberkatalysator nach Anspruch 1 bis 3, dadurch gekennzeichnet, daß die Porenverteilung unimodal oder bimodal ist, wobei im bimodalen Fall die größeren Poren wenigstens 50% des Gesamtporenvolumens betragen und einen mittleren Durchmesser von 10 000 bis 40 000 und die kleineren Poren einen Durchmesser von 500 bis 2000 nm haben und im unimodalen Fall der mittlere Porendurchmesser 1000 bis 5000 nm beträgt.

5. Verfahren zur Herstellung eines Silberkatalysators nach Anspruch 1 bis 4, dadurch gekennzeichnet, daß man als Träger $\alpha$-Al$_2$O$_3$ verwendet und die katalytisch wirksamen Komponenten durch Tränken desselben in einer oder mehreren Stufen mit einer Silbersalzlösung, die komplexbildende Zusätze enthält und Alkalisalzlösungen aufbringt und ebenso in einer oder mehreren Stufen tempert, wobei der Katalysatorträger mit mindestens 13 Gew.-% Silber beaufschlagt wird und eine Silbermasse von mindestens 110 kg Silber pro m³ Reaktor erreicht wird und der Gehalt an Lithium 100 bis 300 Gew.-ppm und der Gehalt an Cäsium 200 bis 600 Gew.-ppm beträgt.

6. Verwendung der Silberkatalysatoren nach Anspruch 1 bis 5 für die Herstellung von Ethylenoxid aus Ethylen und Sauerstoff in der Gasphase.

## Claims

1. A silver catalyst containing lithium and cesium as promoters for the direct oxidation of ethylene with oxygen to give ethylene oxide, on a porous carrier which essentially consists of $\alpha$-alumina and contains certain amounts of soluble calcium, aluminum, potassium and sodium salts, wherein the carrier has a BET surface area of not less than 0.4 m²/g and not more than 0.8 m²/g, a pore volume of not less than 0.45 ml/g, the pores being equally accessible to cold and warm water, a bulk density of not more than 700 kg/m³ and a shape which, in the reactor, provides a geometrical surface area of not less than 600 m²/m³, on which not less than 13% by weight of silver are applied as active component and not less than 110 kg of silver are available per m³ of reactor, and from 100 to 300 ppm by weight of lithium and from 200 to 600 ppm by weight of cesium are present as promoters.

2. A silver catalyst as claimed in claim 1, wherein the carrier contains nitric acid-soluble calcium and/or aluminum compounds in amounts of from 200 to 2000 ppm and not less than 50 ppm of soluble potassium and/or sodium compounds.

3. A silver catalyst as claimed in claim 1 or 2, wherein the carrier material is not less than 99% very pure $\alpha$-Al$_2$O$_3$

4. A silver catalyst as claimed in any of claims 1 to 3, wherein the pore distribution is unimodal or bimodal, in the bimodal case the larger pores accounting for not less than 50% of the total pore volume and having a mean diameter of from 10 000 to 40 000 nm and the smaller pores having a diameter of from 500 to 2 000 nm, and in the unimodal case the mean pore diameter being from 1000 to 5000 nm.

5. A process for preparing a silver catalyst as claimed in any of claims 1 to 4, wherein $\alpha$-Al$_2$O$_3$ is used as the carrier, and the catalytically active components are applied by impregnating the said carrier in one or more stages with a silver salt solution, which contains complex-forming additives, and alkali metal salt solutions and heating the product, likewise in one or more stages, the catalyst carrier being brought into contact with not less than 13% by weight of silver and the resulting mass of silver being not less than 110 kg per m³ of reactor, and the content of lithium being from 100 to 300 ppm by weight and the content of cesium from 200 to 600 ppm by weight.

6. The use of a silver catalyst as claimed in any of claims 1 to 5 for preparing ethylene oxide from ethylene and oxygen in the gas phase.

## Revendications

1. Catalyseur à l'argent, contenant du lithium et du césium comme promoteurs, pour l'oxydation directe d'éthylène en présence d'oxygène, en oxyde d'éthylène, sur un support poreux, constitué pour l'essentiel d'oxyde d'aluminium $\alpha$ et contenant des quantités déterminées de sels solubles de calcium, aluminium, potassium et sodium, caractérisé en ce que le support a une surface BET non inférieure à 0,4 m²/g et non supérieure à 0,8 m²/g, un volume des pores d'au moins 0,45 ml/g, les pores étant accessibles tant à l'eau froide qu'à l'eau chaude, une densité apparente non inférieure à 700 kg/m³ et une forme qui développe dans le réacteur une surface géométrique d'au moins 600 m²/m³, sur laquelle sont déposés au moins 13% en poids d'argent à titre de constituant actif, sont disponibles au moins 110 kg d'argent par m³ de réacteur et sont contenus a titre de promoteurs de 100 à 300 ppm de lithium et de 200 à 600 ppm de césium.

7

2. Catalyseur à l'argent selon la revendication 1, caractérisé en ce que le support comporte des composés de calcium et/ou d'aluminium solubles dans l'acide nitrique à des concentrations de 200 à 2000 ppm et au moins 50 ppm de composés de potassium et/ou de sodium solubles.

3. Catalyseur à l'argent selon les revendications 1 et 2, caractérisé en ce que la matière du support est constituée pour au moins 99% de $\alpha$-Al$_2$O$_3$ de très haute pureté.

4. Catalyseur à l'argent selon les revendications 1 à 3, caractérisé en ce que la distribution des pores est unimodale ou bimodale, où, dans la situation bimodale, les pores les plus grands constituent au moins 50% de l'ensemble du volume des pores et possèdent un diamètre moyen de 10 000 à 40 000 nanomètres et les pores les plus petits ont un diamètre de 500 à 2000 nanomètres, tandis que dans la situation unimodale, le diamètre moyen des pores est de 1000 à 5000 nanomètres.

5. Procédé de préparation d'un catalyseur à l'argent selon les revendications 1 à 4, caractérisé en ce que l'on utilise comme support du $\alpha$-Al$_2$O$_3$ et on dépose les constituants catalytiquement actifs appliqués par imprégnation de ce support, en une ou plusieurs étapes, d'une solution de sel d'argent qui renferme des additifs complexants et qui applique des solutions de sels alcalins et les soumet de même à un traitement thermique en une ou plusieurs étapes, grâce à quoi le support du catalyseur est chargé d'au moins 13% en poids d'argent et on atteint une masse d'argent d'au moins 110 kg d'argent par m$^3$ de réacteur et la teneur en lithium s'élève à 100 à 300 ppm et celle du césium à 200 à 600 ppm.

6. Utilisation des catalyseurs à l'argent selon les revendications 1 à 5 pour l'obtention d'oxyde d'éthylène au départ d'éthylène et d'oxygène en phase gazeuse.